# EUROPEAN PATENT APPLICATION

(11) **EP 1 120 666 A2**
(43) Date of publication of application: **01.08.2001**
(21) Application number: 00311646.4
(22) Date of filing: 22.12.2000
(51) Int. Cl.: G01T 1/29

(54) **Methods and apparatus for variable thickness multi-slice CT imaging**

(30) Priority: 30.12.1999 US 475777
(71) Applicant: GE Medical Systems Global Technology Company LLC, Waukesha, Wisconsin 53188 (US)
(72) Inventor: He, Hui David, Waukesha, Wisconsin 53188 (US); Possin, George Edward, Schenectady, New York 12309 (US); Hoffman, David Michael, New Berlin, Wisconsin 53151 (US); Besson, Guy M., Wauwatosa, Wisconsin 53213 (US); Senzig, Robert, Germantown, Wisconsin 53022 (US); Ackelsberg, Sholom M., Brookfield, Wisconsin 53045 (US)
(74) Representative: Goode, Ian Roy

(57) **Abstract**

The present invention, in one aspect, is a method for collecting images of an object (22) using a multi-slice CT imaging system (10) having a detector array (18) and an x-ray source (14) configured to emit a fan beam of x-rays (16) through an object towards the detector array, the detector array having a plurality of rows (70) of detector elements (20) including rows having different thicknesses. The method includes steps of: passing the fan beam of x-rays through the object; and selectively combining rows of the detector array, including rows having different thicknesses, to obtain data representative of at least one image slice of the object.

## Description

This invention relates generally to methods and apparatus for imaging, and more particularly to methods and apparatus providing variable thickness imaging capability.

In at least one known computed tomography (CT) imaging system configuration, an x-ray source projects a fan-shaped beam which is collimated to lie within an X-Y plane of a Cartesian coordinate system and generally referred to as the "imaging plane". The x-ray beam passes through the object being imaged, such as a patient. The beam, after being attenuated by the object, impinges upon an array of radiation detectors. The intensity of the attenuated beam radiation received at the detector array is dependent upon the attenuation of the x-ray beam by the object. Each detector element of the array produces a separate electrical signal that is a measurement of the beam attenuation at the detector location. The attenuation measurements from all the detectors are acquired separately to produce a transmission profile.

In known third generation CT systems, the x-ray source and the detector array are rotated with a gantry within the imaging plane and around the object to be imaged so that the angle at which the x-ray beam intersects the object constantly changes. A group of x-ray attenuation measurements, i.e., projection data, from the detector array at one gantry angle is referred to as a "view". A "scan" of the object comprises a set of views made at different gantry angles, or view angles, during one revolution of the x-ray source and detector. In an axial scan, the projection data is processed to construct an image that corresponds to a two dimensional slice taken through the object. One method for reconstructing an image from a set of projection data is referred to in the art as the filtered back projection technique. This process converts the attenuation measurements from a scan into integers called "CT numbers" or "Hounsfield units", which are used to control the brightness of a corresponding pixel on a cathode ray tube display.

Known modern multi-slice CT systems make it possible and practical to acquire volumetric scan data and to produce cross-sectional, three-dimensional and reformat images with significantly improved z-axis resolution, i.e., resolution along a patient translation direction. One such system is a scalable, 4-slice system having a detector array of 16 rows of detector elements, each row having equal thickness. (By convention, dimensions along a *z*-axis perpendicular to an imaging plane are referred to herein as "thicknesses." Images of a patient obtained from a CT imaging system represent slices of the patient. Such slices are characterized as representing a volume of a specified thickness.) This system can provide several multi-slice data acquisition modes.

However, to produce three dimensional images with near isotropic spatial resolution, it is necessary to acquire CT scan image data representative of submillimeter slices of a patient. One obvious approach to providing this resolution is to segment the x-ray detection system to submillimeter dimensions. However, doing so would significantly increase the overall complexity and cost of device fabrication, particularly if the thicker slice capability of known imaging system is also to be retained. In addition, x-ray detection quantum efficiency would be adversely affected by such small detector segmentation. It would therefore be desirable to provide methods and apparatus for detecting multiple image slices including submillimeter slices without sacrificing the ability to obtain thicker image slices including the same region imaged as submillimeter slices. It would also be desirable to provide methods and apparatus for providing a wider selection of slice thicknesses than is available utilizing equally-segmented detector arrays.

There is therefore provided, in one aspect of the present invention, a method for collecting images of an object using a multi-slice CT imaging system having a detector array and an x-ray source configured to emit a fan beam of x-rays through an object towards the detector array, the detector array having a plurality of rows of detector elements including rows having different thicknesses. The method includes steps of: passing the fan beam of x-rays through the object; and selectively combining rows of the detector array, including rows having different thicknesses, to obtain data representative of at least one image slice of the object.

The above-described embodiment can provide, among other advantages, a greater selection of slice thicknesses than is available with methods utilizing equally-segmented detector arrays having the same number of segments in a *z*-axis direction. The selection of slice thicknesses can include submillimeter slice thicknesses.

Embodiments of the invention will now be described, by way of example, with reference to the accompanying drawings, in which:
Figure 1 is a pictorial view of a CT imaging system.
Figure 2 is a block schematic diagram of the system illustrated in Figure 1.
Figure 3 is a perspective view of a CT system detector array.
Figure 4 is a perspective view of one of the detector modules of the detector array shown in Figure 3.
Figure 5 is illustrative of various configurations of the detector module of Figure 4 in a four-slice mode.
Figure 6 is an illustrative cross-sectional side view of a multi-slice detector array having non-equal row thicknesses and in which each detector element has one photodiode and one scintillator cell having a thickness equal to the associated photodiode.
Figure 7 is an illustrative top view of a scintillator array of a detector module suitable for the multi-slice detector array shown in Figure 6.
Figure 8 is a simplified side view illustration showing relative orientation and positioning of the rows of the detector array of Figure 6 with respect to the x-ray source, table and patient shown in Figures 1 and 2, as used in a CT imaging system.
Figure 9 is an illustrative cross-sectional side view of a multi-slice detector array having non-equal row thicknesses and in which each detector element has one scintillator cell and one or more diodes hardwired to produce a single output representative of scintillation from the associated scintillator cell.
Figure 10 is an illustrative cross-sectional side view of a multi-slice detector array having non-equal row thicknesses, wherein the rows are produced by hardwiring sets of photodiodes together.

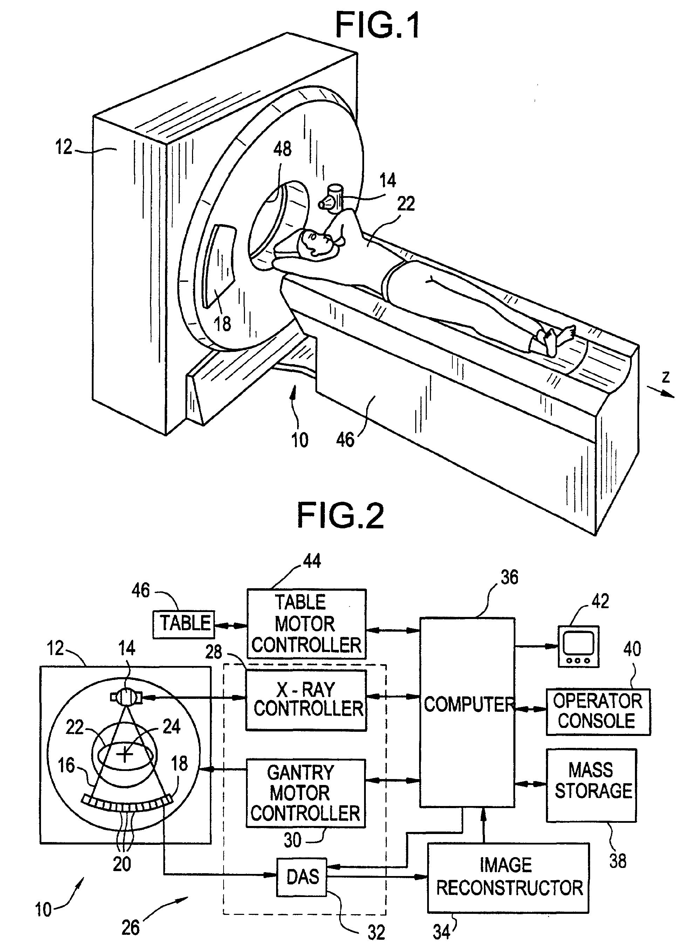

Referring to Figures 1 and 2, a computed tomograph (CT) imaging system 10 is shown as including a gantry 12 representative of a "third generation" CT scanner. Gantry 12 has an x-ray source 14 that projects a beam of x-rays 16 toward a detector array 18 on the opposite side of gantry 12. Detector array 18 is formed by detector elements 20 which together sense the projected x-rays that pass through an object 22, for example a medical patient. Detector array 18 may be fabricated in a single slice or multi-slice configuration. Each detector element 20 produces an electrical signal that represents the intensity of an impinging x-ray beam and hence the attenuation of the beam as it passes through patient 22. During a scan to acquire x-ray projection data, gantry 12 and the components mounted thereon rotate about a center of rotation or isocenter 24.

Rotation of gantry 12 and the operation of x-ray source 14 are governed by a control mechanism 26 of CT system 10. Control mechanism 26 includes an x-ray controller 28 that provides power and timing signals to x-ray source 14 and a gantry motor controller 30 that controls the rotational speed and position of gantry 12. A data acquisition system (DAS) 32 in control mechanism 26 samples analog data from detector elements 20 and converts the data to digital signals for subsequent processing. An image reconstructor 34 receives sampled and digitized x-ray data from DAS 32 and performs high speed image reconstruction. The reconstructed image is applied as an input to a computer 36 which stores the image in a mass storage device 38.

Computer 36 also receives commands and scanning parameters from an operator via console 40 that has a keyboard. An associated cathode ray tube display 42 allows the operator to observe the reconstructed image and other data from computer 36. The operator supplied commands and parameters are used by computer 36 to provide control signals and information to DAS 32, x-ray controller 28 and gantry motor controller 30. In addition, computer 36 operates a table motor controller 44 which controls a motorized table 46 to position patient 22 in gantry 12. Particularly, table 46 moves portions of patient 22 through gantry opening 48.

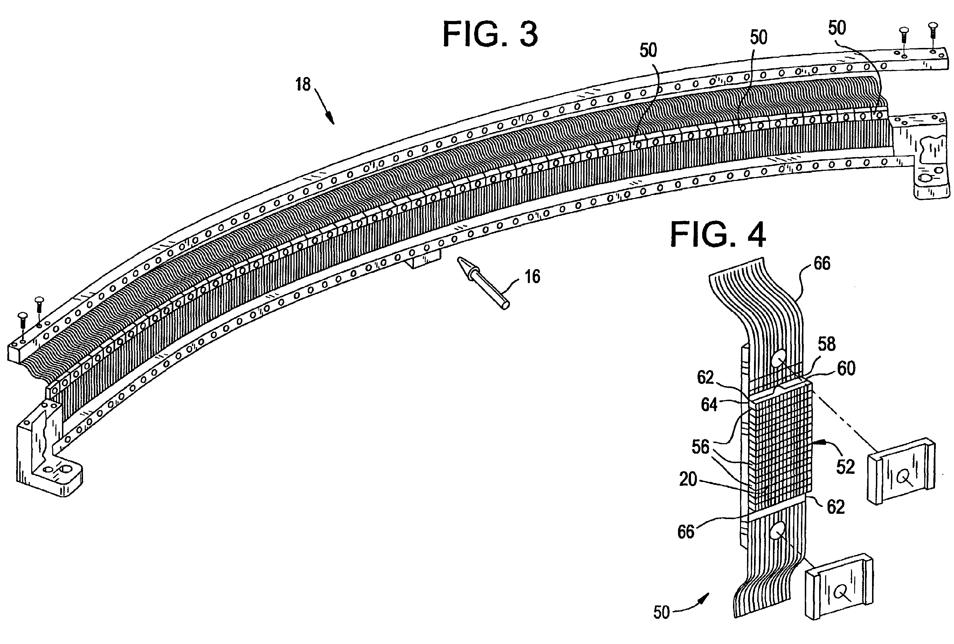

As shown in Figures 3 and 4, detector array 18 includes a plurality of detector modules 50, each module comprising an array of detector elements 20. Each detector module 50 includes a high-density semiconductor sensing array 52 and a multidimensional scintillator array 54 positioned above and adjacent to semiconductor array 52. A collimator (not shown in Figure 3 or 4) is positioned above and adjacent scintillator array 54 to collimate x-ray beams 16 before such beams impinge upon scintillator array 54. Particularly, semiconductor array 52 includes a plurality of photodiodes 56, a switch array 58, and a decoder 60. In one embodiment, switch array 58 and decoder 60 are fabricated on one or more device structures that are denoted herein as "switch apparatus." In the embodiment illustrated in Figure 3, there are two switch apparatus 64 and 66 at opposite ends of module 50. Scintillator array 54, as known in the art, is positioned over adjacent photodiodes 56. Photodiodes 56 are optically coupled to scintillator array 54 and have electrical output lines 62 for transmitting signals representative of the light output by scintillator array 54. Each photodiode 56 produces a separate low level analog output signal that is a measurement of beam attenuation for a specific scintillator of scintillator array 54. Photodiode output lines 62 may, for example, be physically located on one side of module 50 or on a plurality of sides of module 50. As shown in Figure 4, photodiode outputs 62 are located at opposing sides of the photodiode array.

In one embodiment, as shown in Figure 3, detector array 18 includes fifty-seven detector modules 50. Each detector module 50 includes an array of detector elements 20 comprising, in part, a semiconductor array 52 and a scintillator array 54. One embodiment includes an array of 16 x 16 detector elements 20 in each module. As a result, array 18 is segmented into 16 rows and 912 columns (16 x 57 modules).

Switch apparatus 64 is coupled between semiconductor array 52 and DAS 32. One embodiment includes two semiconductor switch apparatus 64 and 66. Switch apparatus 64 and 66 each include a plurality of field effect transistors (FETs). Each FET includes an input electrically connected to one of the respective photodiode output lines 62, an output, and a control. FET outputs and controls are connected to lines that are electrically connected to DAS 32 via a flexible electrical cable 68. Particularly, about one-half of photodiode output lines 62 are electrically connected to each FET input line of switch apparatus 64 with the other one-half of photodiode output lines 62 electrically connected to FET input lines of switch apparatus 66.

Decoder 60 controls the operation of switch array 58 to enable, disable, or combine photodiode 56 outputs depending upon a desired number of slices and slice resolutions for each slice. Decoder 60, in one embodiment, is a digital logic circuit as known in the art. Decoder 60 includes a plurality of output and control lines coupled to switch array 58 and DAS 32. Particularly, the decoder outputs are electrically coupled to the switch apparatus control lines to enable switch array 58 to transmit the photodiode signals from the switch array inputs to the switch array outputs. Utilizing decoder 60, specific FETs within switch array 58 are selectively enabled, disabled, or combined so that specific photodiode 56 outputs are electrically connected to CT system DAS 32. In one embodiment, decoder 60 controls switch array 58 so that a selected number of rows of semiconductor array 52 are connected to DAS 32, resulting in a selected number of slices of data being electrically connected to DAS 32 for processing.

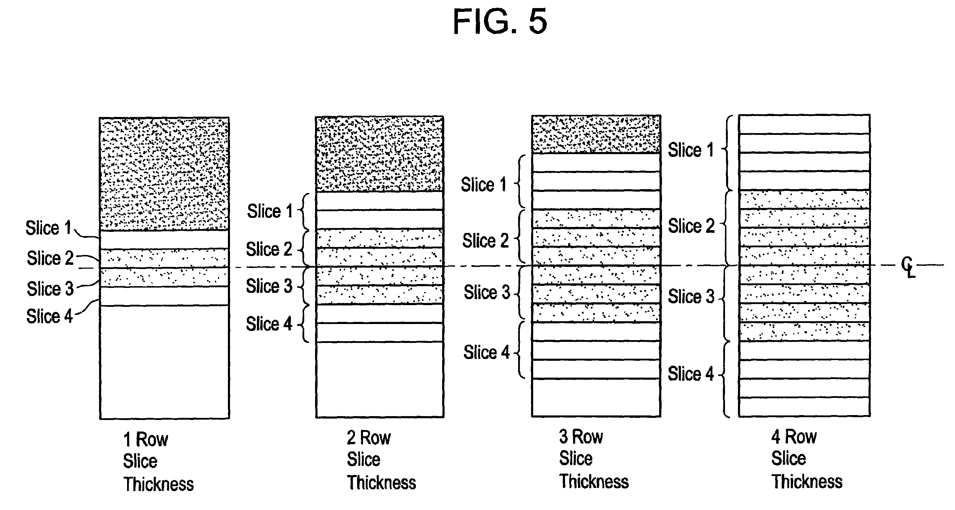

For example, decoder 60 selects from modes including one, two, and four slice modes. As shown in Figure 5, by transmitting the appropriate decoder control lines, switch apparatus 58 is configurable for a four slice mode in which data is collected from four slices, each comprising one or more rows of semiconductor photodiode array 52. Depending upon a selected configuration of switch apparatus 58 defined by decoder control lines, various combinations of photodiode 56 outputs are selectively enabled, disabled, or combined so that the slice thicknesses consist of 1, 2, 3, or 4 rows of photodiode array elements. In one embodiment, switch apparatus 58 and decoder 60 are combined into a single semiconductor chip.

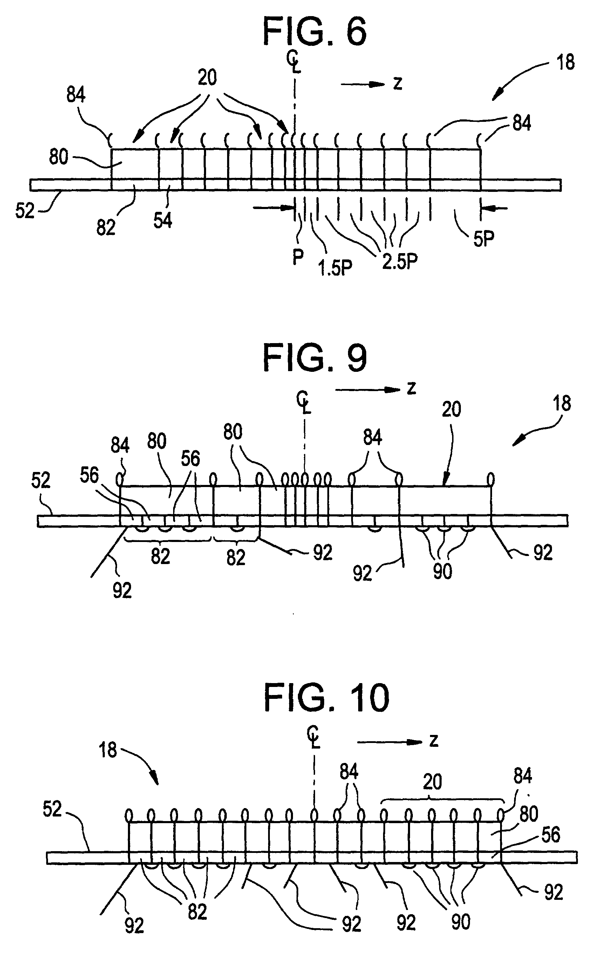

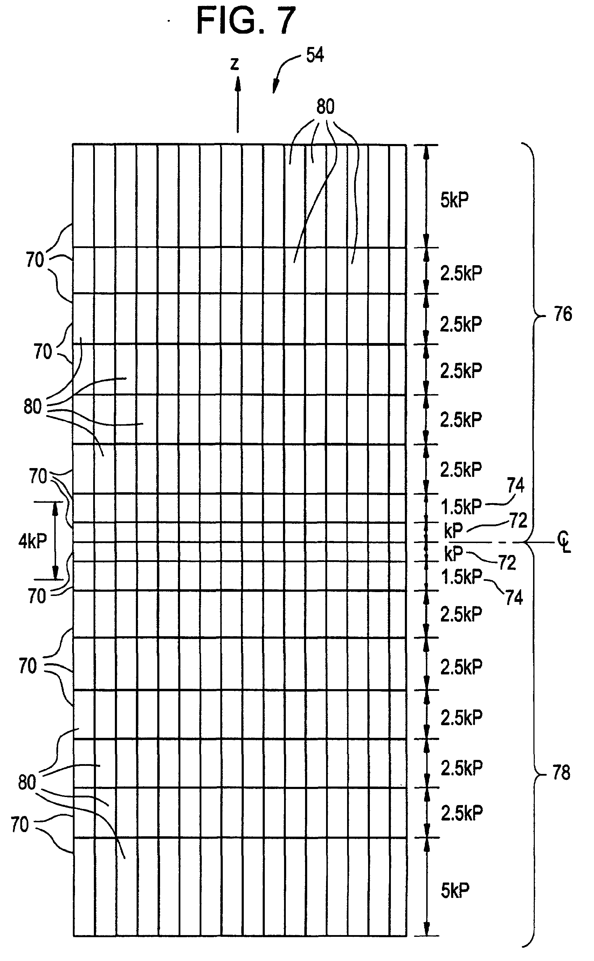

In one embodiment as illustrated in Figures 6, 7 and 8, a detector array 18 is provided having *N*=16 rows 70 of detector elements 20 extending along a *z*-axis, wherein the *N* rows include rows 72, 74 that do not have equal thicknesses in the *z*-axis direction. The *N* rows 70 are arranged as mirror image sets of *N*/2 rows 76, 78 on each side of a centerline CL of detector array 18, with rows at increasing distance from centerline CL having non-decreasing thicknesses. Each detector element 20 of detector array 18 in this embodiment comprises one scintillator cell 80, a photodiode cell 82 having a thickness in the *z*-axis direction that is equal to that of scintillator cell 80, and an x-ray collimator 84. Photodiode cell 82 is configured to detect scintillations exclusively from scintillator cell 80 that is in the same detector element 20. Photodiode cells 82 each comprise exactly one photodiode 56, and not all photodiodes 56 have the same thicknesses. On both sides of a centerline CL of detector array 18, detector cells 20 are arranged in rows having thicknesses *k*P, 1.5*k*P, 2.5*k*P, 2.5*k*P, 2.5*k*P, 2.5*k*P, 2.5*k*P and 5*k*P, where *k* and P are defined below. Thus, the rows have thickness ratios of 1 : 1.5 : 2.5 : 2.5 : 2.5 : 2.5 : 2.5 : 5 in both directions from centerline CL. A top view of an embodiment of a scintillator array 54 of a detector module 50 suitable for use in this detector array 18 embodiment is shown in Figure 7.

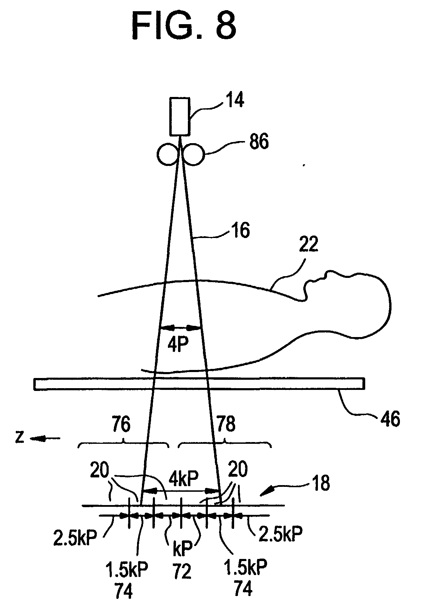

As used herein, P is an effective slice thickness that an image of patient 22 would have if taken utilizing a fan beam 16 impinging on exactly a thickness *k*P of detector array 18, where *k* is a constant dependent upon geometry. Thickness P is a design choice selected in accordance with imaging requirements, for example, 0.5 mm or another thickness between 0.2 and 2.0 mm. These values are merely exemplary, however, as the invention is applicable to values of P outside this range. Because patient 22 is between x-ray source 14 and detector array 18, fan beam 16 spreads, as shown in Figure 8, and is somewhat thicker than the effective slice thickness when it impinges detector array 18. Therefore, proportionality constant *k* is greater than 1.0 (for example, 1.7), and is dependent upon geometry.

A pre-patient collimator 86 is used in some operating modes of a detector 18 embodiment having thickness ratios of 1 : 1.5 : 2.5 : 2.5 : 2.5 : 2.5 : 2.5 : 5. For example, to obtain four image slices each having an effective thickness P, pre-patient collimator 86 is used to limit a thickness of fan beam 16 a thickness 4P referenced to patient 22. Fan beam 16 therefore impinges only central rows of detector 18 having thicknesses 1.5*k*P, *k*P, *k*P, and 1.5*k*P, respectively. However, because fan beam 16 is constrained by pre-patient collimation 86, it has a thickness of only 4*k*P at detector 18. Therefore, only 1.0*k*P of the 1.5*k*P detector rows is actually impinged by x-rays. Detector 18 thus effectively provides four rows of 1.0*k*P slices in this mode of operation.

In other modes of operation, outputs from adjacent photodiode cells 82 along the z-axis are selectively combined to obtain image slices having other selected thicknesses. When P is on the order of one millimeter, the progressive segmentation of scintillator cells 80 outward along the *z*-axis from the center of detector array 18 provides modes of operation having, for example, one, two, or four slices having an advantageously wide range of thicknesses. For example, a 16-row detector 18 embodiment having thickness ratios of 1 : 1.5 : 2.5 : 2.5 : 2.5 : 2.5 : 2.5 : 5 provides imaging system 10 with a selection of multi-slice imaging modes covering a much wider range of thicknesses than a detector array having only rows of equal thickness. This wider selection is achieved without significant increases in data acquisition, detector/DAS interconnection and data transfer hardware. Examples of Z-axis slice thicknesses that are selectively obtained utilizing this embodiment of detector array 18 with P=0.5 mm (in some cases, utilizing pre-patient collimation) are: 0.5 mm, 1 mm, 1.25 mm, 2 mm, 2.5 mm, 3.75 mm, 5 mm, 7.5 mm, and 10 mm.

In one embodiment, a number of datasets representing image slices are selected in accordance with input channel limitations of DAS 32. Table I shows how multiple axial step-and-shoot CT images of 0.5 mm, 1.0 mm, 1.25 mm, 2 mm, 2.5 mm, 3.75 mm, and 5 mm are selectively generated simultaneously per gantry rotation by combining adjacent scintillator rows 88 selectively along the *z*-axis direction. Relative efficiency loss is minimized due to use of elements with appropriate pre-patient collimation to reduce an effective thickness of the outer slices in some data acquisition modes.

**TABLE I**

| MM Slice | Scintillator Cells Used |
|---|---|
| 0.5 mm | 4 slices: (1.5*k*P), (*k*P), (*k*P), (1.5*k*P) with outer rows limited by collimation |
| | |
| 1 mm | 2 slices: (1.5kP, *k*P), (*k*P, 1.5*k*P) with outer rows limited by collimation |
| | |
| 1.25 mm | 2 slices: (1.5*k*P, *k*P), (*k*P, 1.5*k*P) |
| | |
| 2 mm | 1 slice: (1.5*k*P, *k*P, *k*P, 1.5*k*P) with outer rows limited by collimation |
| | |
| 2 mm (alternate) | 2 slices: (2.5*k*P, 1.5*k*P), (1.5*k*P, 2.5*k*P) (central *k*P, *k*P omitted) |
| | |
| 2.5 mm | 8 slices: (5*k*P), (2.5*k*P, 2.5*k*P), (2.5*k*P, 2.5*k*P), (2.5*k*P, 1.5*k*P, *k*P), (*k*P, 1.5*k*P, 2.5*k*P), (2.5*k*P, 2.5*k*P), (2.5*k*P, 2.5*k*P), (5*k*P) |
| | |
| 3.75 mm | 4 slices: (2.5*k*P, 2.5*k*P, 2.5*k*P), (2.5*k*P, 2.5*k*P, 1.5*k*P, *k*P), (*k*P, 1.5*k*P, 2.5*k*P, 2.5*k*P), (2.5*k*P, 2.5*k*P, 2.5*k*P) |
| | |
| 5 mm | 4 slices: (5*k*P, 2.5*k*P, 2.5*k*P), (2.5*k*P, 2.5*k*P, 2.5*k*P, 1.5*k*P, *k*P), (*k*P, 1.5*k*P, 2.5*k*P, 2.5*k*P, 2.5*k*P), (2.5*k*P, 2.5*k*P, 5*k*P) |

The detector array 18 embodiment described above should be considered as being exemplary of an embodiment having a plurality of rows of detector elements, wherein not all rows have equal thickness. For example, in another embodiment of detector array 18, relative row thicknesses are *k*P, *k*P, 2*k*P, 2*k*P, 2*k*P, 2*k*P, 2*k*P, 4*k*P (corresponding to thickness ratios of 1 : 1 : 2 : 2 : 2 : 2 : 2 : 4). In yet another embodiment, the relative thicknesses are *k*P, *k*P, 2*k*P, 2*k*P, 2*k*P, 2*k*P, 10*k*P, 20*k*P (corresponding to thickness ratios of 1 : 1 : 2 : 2 : 2 : 2 : 10 : 20). Of course, image slice thicknesses obtainable from, and row combinations useful with detector arrays 18 having different sets of row thicknesses will, in general, differ between embodiments. However, in some embodiments, rows 70 of detector array 18 are selected for providing adjacent groupings of adjacent rows of detector elements 20, each of the adjacent groupings having equal thickness, but not all groupings having the same numbers of rows of detector elements 20. Groupings contained in Table I are illustrative of such groupings for a particular embodiment of detector array 18. Pre-patient collimation 86 that selectively limits x-ray impingement to a portion of a detector row (e.g., detector row 74) less than its physical width is not required in all detector array 18 embodiments, and is used in some embodiments only for a limited number of modes.

In one embodiment, detector array 18 has rows 70 of detector elements 20 that include a binary thickness sequence that increases in thickness as a distance from centerline CL increases. Rather than four central rows 70 of thickness 1.5*k*P, *k*P, *k*P, 1.5*k*P as in Figures 6 and 7, these central rows have equal thickness *k*P. Each row 70 further away from the center than the four central rows has twice the thickness of the preceding row. Thus, in one embodiment, *N*=16 and the eight rows on each side of a centerline CL of detector array 18 have thicknesses *k*P, *k*P, 2*k*P, 4*k*P, 8*k*P, 16*k*P, 32*k*P, 64*k*P in each direction from centerline CL, corresponding to thickness ratios of 1 : 1 : 2 : 4 : 8 : 16 : 32 : 64. Four image slices of equal thickness are obtained by combining M detector rows(s) on each side of the center of detector array 18 to obtain two inner aggregate slices each having thickness 2^{*M*}⁻¹*k*P. The two immediately adjacent rows surrounding the inner M detector row(s) are utilized for the two other slices. It will be observed that the adjacent rows also have thickness 2^{*M*}⁻¹*k*P. Thus, when scanning four equal slices at a time in a multi-slice mode, image thicknesses from P to 64P are selectable. Similarly, image thicknesses from P to 128P are selectable in a two-slice mode, and from P to 256P in single-slice mode. In general, the binary sequence ensures that a selected plurality of adjacent rows 70 starting at centerline CL equals a thickness of a next adjacent row 70.

In one embodiment and referring to Figure 9, *N* rows are provided in detector array 18 utilizing scintillator cells 80 and collimators 84 having different thicknesses. In this embodiment, some detector elements 20 have photodiode cells 82 comprising fixed-thickness photodiodes 56 that have hard-wired connections 90 between them. Thus, photodiode cells 82 match a thickness of a corresponding, associated scintillator cell 80, and in doing so, produce a single output 92 representative of scintillation produced exclusively by the corresponding, associated scintillator cell 80. This embodiment has an advantage of ensuring that all photodiode cells 82 have similar optical and thermal response.

In one embodiment and referring to Figure 10, *N* rows are provided by scintillator cells 80, collimators 84, and photodiode cells 82 all having the same thickness. Each photodiode cell 82 corresponds to a scintillator cell 80. For some of the *N* rows, a plurality of photodiode cells 82 are interconnected by hardwired connections 90 in a *z*-axis direction so that a single output 92 is provided that is representative of scintillation produced by a corresponding equal plurality of scintillator cells 80. This embodiment has an advantage of ensuring that all photodiode cells 82 have similar optical and thermal response. In addition, this embodiment can be produced readily from embodiments of detector array 18 that otherwise have equal thickness rows 70 by modifying high-density semiconductor array 52.

In one embodiment, images of an object are collected utilizing a multi-slice CT imaging system 10 having a detector array 18 and an x-ray source 14 configured to emit a fan beam of x-rays 16 through an object 22 towards detector array 18. Detector array 18 has a plurality of rows 70 of detector elements 20 including rows 72, 74 having different thicknesses. The images are obtained by passing fan beam 16 through object 18 and selectively combining rows 70 of detector array 18 impinged by the fan beam of x-rays 16, including rows 72, 74 having different thicknesses, to obtain data representative of at least one image slice of the object. In a variation of this embodiment, the selective combination of rows 70 of detector array 18 includes the selective combination of rows of the detector array into groups (for example, those of Table I) having equal thicknesses, but not all including the same numbers of rows, to obtain data corresponding to each group of rows, each group of rows corresponding to an image slice.

In one embodiment, images of an object are collected utilizing a multi-slice CT imaging system 10 having a detector array 18 and an x-ray source 14 configured to emit a fan beam of x-rays 16 through an object 22 towards detector array 18. CT imaging system 10 also includes a pre-patient collimator 86 configured to limit the thickness of fan beam 16. Detector array 18 has a plurality of rows 70 of detector elements 20 including rows 72, 74 having different thicknesses. Images are collected by selectively limiting the thickness of x-ray beam 16 so that at least one row (e.g., row 74 in Figure 6) of detector array 18 is only partially impinged by fan beam 16. The limited thickness fan beam 16 is passed through object 22. Data from detector array 18 is collected, including data representative of the at least one row 74 of detector array 18 that is only partially impinged by fan beam 16. The thickness of x-ray beam 16 is selected so that data representative of a plurality of image slices, including a slice having data from row 74, is representative of equal thickness image slices.

Although detector array 18 embodiments described in detail herein have *N*=16 rows, the invention is applicable to detector array 18 embodiments having a greater or smaller number of rows 70. For example, embodiments having *N*>16 have more than eight rows of detector cells on either side of a centerline. Such embodiments include, but are not limited to, those having rows having a thickness ratio sequence starting with the same sequence as one of the embodiments described herein having *N*=16, with additional rows of detector cells extending beyond the 16 central rows. The invention is not limited, however, to *N*≥16, or solely to specific thickness ratio patterns described herein.

From the preceding description, it is evident that z-axis resolution (i.e., slice thickness) and scan coverage is improved in some embodiments without substantial additional component complexity or additional cost. In some embodiments, additional data acquisition flexibility is provided that can be used advantageously in clinical applications. Some embodiments provide both advantages.

Although the invention has been described and illustrated in detail, it is to be clearly understood that the same is intended by way of illustration and example only and is not to be taken by way of limitation. In addition, the CT system described herein is a "third generation" system in which both the x-ray source and detector rotate with the gantry. Many other CT systems including "fourth generation" systems wherein the detector is a full-ring stationary detector and only the x-ray source rotates with the gantry, may be used if individual detector elements are corrected to provide substantially uniform responses to a given x-ray beam. Also, the present invention may be used with systems performing either axial or helical scans, or both types of scans.

For completeness, various aspects of the invention are set out in the following numbered clauses:
1. A method for collecting images of an object using a multi-slice CT imaging system having a detector array and an x-ray source configured to emit a fan beam of x-rays through an object towards the detector array, the detector array having a plurality of rows of detector elements including rows having different thicknesses, said method comprising:
   passing the fan beam of x-rays through the object; and
   selectively combining rows of the detector array, including rows having different thicknesses, to obtain data representative of at least one image slice of the object.
2. A method in accordance with Clause 1 wherein said step of selectively combining rows of the detector array comprises the steps of selectively combining rows of the detector array into a plurality of groups having equal thicknesses, the groups not all including the same number of rows, to obtain data corresponding to each group of rows, each group of rows corresponding to an image slice of the object.
3. A method for collecting images of an object using a multi-slice CT imaging system having a detector array, an x-ray source configured to emit a fan beam of x-rays through an object towards the detector array, and a pre-patient collimator configured to limit a thickness of the x-ray beam, the detector array having a plurality of rows of detector elements including rows having different thicknesses, said method comprising:
   selectively limiting the thickness of the x-ray beam so that at least one row of the detector array is only partially impinged by the fan beam of x-rays;
   passing the fan beam of x-rays through the object; and
   collecting image data of the object including image data from a plurality of rows of the detector array, including the at least one row of the detector array that is only partially impinged by the fan beam of x-rays.
4. A method in accordance with Clause 3 wherein collecting image data of the object comprises collecting data representative of a plurality of image slices, at least one of the image slices including data representative of the at least one row of the detector array that is only partially impinged by the fan beam of x-rays, and wherein selectively limiting the thickness of the x-ray beam comprises the step of selecting a thickness of the x-ray beam so that data representative of a plurality of image slices, including a slice having data from the partially impinged detector row, is representative of equal thickness image slices.
5. A detector array for a multi-slice imaging system, said detector array comprising a plurality of rows of detector elements wherein not all of said rows have equal thickness.
6. A detector array in accordance with Clause 5 wherein said rows have thicknesses selected for providing adjacent groupings of adjacent rows, the adjacent groupings having equal thickness and not all of the groupings having equal numbers of rows.
7. A detector array in accordance with Clause 5 having *N* rows arranged in mirror image sets of *N*/2 slices about a centerline of said detector array, each said set having rows arranged in an order having non-decreasing thicknesses as a distance from the centerline of said detector array.
8. A detector array in accordance with Clause 7 wherein each set of *N/2* rows includes rows having a binary sequence of increasing thicknesses as a distance from the centerline increases, so that a sum of the thicknesses of a selected plurality of adjacent rows starting at the centerline equals a thickness of a next adjacent row.
9. A detector array in accordance with Clause 8 wherein rows in each set of *N*/2 rows have a sequence of thickness ratios beginning 1 : 1 : 2 : 4 : 8 : 16 : 32, starting at the centerline.
10. A detector array in accordance with Clause 7 wherein rows in each set of *N*/2 rows have a sequence of thickness ratios beginning 1 : 1.5 : 2.5 : 2.5 : 2.5 : 2.5 : 2.5 : 5, starting at the centerline.
11. A detector array in accordance with Clause 7 wherein rows in each set of *N*/2 rows have a sequence of thickness ratios beginning 1 : 1 : 2 : 2 : 2 : 2 : 2 : 4, starting at the centerline.
12. A detector array in accordance with Clause 7 wherein rows in each set of *N*/2 rows have a sequence of thickness ratios beginning 1 : 1 : 2 : 2 : 2 : 2 : 10 : 20, starting at the centerline.
13. A detector array in accordance with Clause 5 wherein each said detector element comprises one photodiode cell and one associated scintillator cell, and said photodiode cell is configured to detect scintillation exclusively from said associated scintillator cell.
14. A detector array in accordance with Clause 13 wherein said photodiode cell includes exactly one photodiode.
15. A detector array in accordance with Clause 13 wherein said photodiode cell includes a plurality of photodiodes interconnected to produce a single output representative of scintillation from said associated scintillator cell.
16. A detector array in accordance with Clause 13 wherein each said detector element comprises a plurality of photodiode cells and an equal plurality of associated scintillator cells, and the plurality of photodiode cells are interconnected to produce a single output representative of scintillation produced by said associated equal plurality of scintillator cells.
17. A CT imaging system comprising a detector array in accordance with Clause 5 and an x-ray source configured to emit a fan beam of x-rays towards said detector array through an object between said x-ray source and said detector array, said CT imaging system also being selectively configurable to combine groupings of adjacent rows of said detector array for acquiring data representative of an image slice.
18. A CT imaging system in accordance with Clause 17 wherein said rows of said detector array have thicknesses selected for providing adjacent groupings of adjacent rows, said adjacent groupings having equal thickness and not all of the groupings having equal numbers of rows, and wherein said CT imaging system is a multi-slice imaging system selectively configurable to select said adjacent groupings for acquiring of data representative of a plurality of image slices during a scan.
19. A CT imaging system in accordance with Clause 17 wherein said detector array has *N* rows arranged in mirror image sets of *N*/2 slices about a centerline of said detector array, each said set having rows arranged in an order having non-decreasing thicknesses as a distance from the centerline of said detector array.
20. A CT imaging system in accordance with Clause 19 wherein each set of *N*/2 rows includes rows having a binary sequence of increasing thicknesses as a distance from the centerline of said detector array increases, so that a sum of the thicknesses of a selected plurality of adjacent rows starting at the centerline equals a thickness of a next adjacent row.
21. A CT imaging system in accordance with Clause 20 wherein rows in each set of *N*/2 rows have a sequence of thickness ratios beginning 1 : 1 : 2 : 4 : 8 : 16 : 32, starting at the centerline of said detector array.
22. A CT imaging system in accordance with Clause 19 wherein rows in each set of *N*/2 rows have a sequence of thickness ratios beginning 1 : 1.5 : 2.5 : 2.5 : 2.5 : 2.5 : 2.5 : 5, starting at the centerline of said detector array.
23. A CT imaging system in accordance with Clause 19 wherein rows in each set of *N*/2 rows have a sequence of thickness ratios beginning 1 : 1 : 2 : 2 : 2 : 2 : 2 : 4, starting at the centerline of said detector array.
24. A CT imaging system in accordance with Clause 19 wherein rows in each set of *N*/2 rows have a sequence of thickness ratios beginning 1 : 1 : 2 : 2 : 2 : 2 : 10 : 20, starting at the centerline of said detector array.
25. A CT imaging system in accordance with Clause 17 wherein each said detector element comprises one photodiode cell and one associated scintillator cell, and said photodiode cell is configured to detect scintillation exclusively from said associated scintillator cell.
26. A CT imaging system in accordance with Clause 25 wherein said photodiode cell includes exactly one photodiode.
27. A CT imaging system in accordance with Clause 25 wherein said photodiode cell includes a plurality of photodiodes interconnected to produce a single output representative of scintillation from said associated scintillator cell.
28. A CT imaging system in accordance with Clause 25 wherein each said detector element comprises a plurality of photodiode cells and an equal plurality of associated scintillator cells, and the plurality of photodiode cells are interconnected to produce a single output representative of scintillation produced by said associated equal plurality of scintillator cells.
29. A CT imaging system in accordance with Clause 17 further comprising a pre-patient collimator configured to selectively limit a thickness of the fan beam of x-rays.

## Claims

1. A method for collecting images of an object using a multi-slice CT imaging system (10) having a detector array (18) and an x-ray source (14) configured to emit a fan beam of x-rays (16) through an object (22) towards the detector array, the detector array having a plurality of rows of detector elements (70) including rows having different thicknesses, said method comprising:
passing the fan beam of x-rays through the object; and
selectively combining rows of the detector array, including rows having different thicknesses, to obtain data representative of at least one image slice of the object.

2. A method in accordance with Claim 1 wherein said step of selectively combining rows (70) of the detector array (18) comprises the steps of selectively combining rows of the detector array into a plurality of groups having equal thicknesses, the groups not all including the same number of rows, to obtain data corresponding to each group of rows, each group of rows corresponding to an image slice of the object (22).

3. A method for collecting images of an object (22) using a multi-slice CT imaging system (10) having a detector array (18), an x-ray source (14) configured to emit a fan beam of x-rays (16) through an object towards the detector array, and a pre-patient collimator (86) configured to limit a thickness of the x-ray beam, the detector array having a plurality of rows (70) of detector elements (20) including rows having different thicknesses, said method comprising:
selectively limiting the thickness of the x-ray beam so that at least one row of the detector array is only partially impinged by the fan beam of x-rays;
passing the fan beam of x-rays through the object; and
collecting image data of the object including image data from a plurality of rows of the detector array, including the at least one row of the detector array that is only partially impinged by the fan beam of x-rays.

4. A method in accordance with Claim 3 wherein collecting image data of the object (22) comprises collecting data representative of a plurality of image slices, at least one of the image slices including data representative of the at least one row of the detector array (18) that is only partially impinged by the fan beam of x-rays (16), and wherein selectively limiting the thickness of the x-ray beam comprises the step of selecting a thickness of the x-ray beam so that data representative of a plurality of image slices, including a slice having data from the partially impinged detector row, is representative of equal thickness image slices.

5. A detector array (18) for a multi-slice imaging system (10), said detector array comprising a plurality of rows (70) of detector elements (20) wherein not all of said rows have equal thickness.

6. A detector array (18) in accordance with Claim 5 wherein said rows (70) have thicknesses selected for providing adjacent groupings of adjacent rows, the adjacent groupings having equal thickness and not all of the groupings having equal numbers of rows.

7. A detector array (18) in accordance with Claim 5 having N rows (70) arranged in mirror image sets of *N*/2 (76, 78) slices about a centerline of said detector array, each said set having rows arranged in an order having non-decreasing thicknesses as a distance from the centerline of said detector array.

8. A CT imaging system (10) comprising a detector array (18) in accordance with Claim 5 and an x-ray source (14) configured to emit a fan beam of x-rays (16) towards said detector array through an object (22) between said x-ray source and said detector array, said CT imaging system also being selectively configurable to combine groupings of adjacent rows (70) of said detector array for acquiring data representative of an image slice.

9. A CT imaging system (10) in accordance with Claim 8 wherein said rows (70) of said detector array (18) have thicknesses selected for providing adjacent groupings of adjacent rows, said adjacent groupings having equal thickness and not all of the groupings having equal numbers of rows, and wherein said CT imaging system is a multi-slice imaging system selectively configurable to select said adjacent groupings for acquiring of data representative of a plurality of image slices during a scan.

10. A CT imaging system (10) in accordance with Claim 8 wherein said detector array (18) has *N* rows (70) arranged in mirror image sets of *N*/2 *(76,* 78) slices about a centerline of said detector array, each said set having rows arranged in an order having non-decreasing thicknesses as a distance from the centerline of said detector array.
